# EUROPÄISCHE PATENTSCHRIFT

(11) **EP 2 245 990 B1**
(45) Veröffentlichungstag und Bekanntmachung des Hinweises auf die Patenterteilung: **21.10.2015**
(21) Anmeldenummer: 10004247.2
(22) Anmeldetag: 21.04.2010
(51) Int. Cl.: A61B 17/02, A61B 17/24

(54) **Medizinisches Instrument zum Dilatieren von knöchernen Strukturen**
Medical instrument for dilating bone structures
Instrument médical pour la dilatation de structures osseuses

(30) Priorität: 27.04.2009 DE 102009018723
(43) Veröffentlichungstag der Anmeldung: 03.11.2010
(73) Patentinhaber: Karl Storz GmbH & Co. KG, 78532 Tuttlingen (DE)
(72) Erfinder: Stammberger, Heinz Prof. Dr., 8043 Graz (AT); Blocher, Martin, 78532 Tuttlingen (DE)
(74) Vertreter: Hofmeister, Frank

(56) Entgegenhaltungen:
- WO-A1-99/17661
- WO-A2-2008/042155
- WO-A2-2008/134288
- US-A1- 2003 023 260
- US-A1- 2009 024 158

## Beschreibung

Die Erfindung betrifft ein medizinisches Instrument zum Dilatieren von knöchernen Strukturen, mit einem Schaft, an dessen distalem Ende eine Werkzeugspitze angeordnet ist und an dessen proximalem Ende eine Handhabe angeordnet ist, wobei die Handhabe und die Werkzeugspitze über ein Betätigungselement derart miteinander in Wirkverbindung stehen, dass durch Betätigen der Handhabe die Werkzeugspitze zumindest teilweise radial erweiterbar ist und wobei das distale Ende des Betätigungselements verdickt ausgebildet ist.

Erfindungsgemäße Dilatationsinstrumente werden beispielsweise in der endoskopischen HNO-Chirurgie verwendet, um die Stirnhöhlen eines Patienten zu erweitern und knöcherne Verwachsungen in der Stirnhöhle zu entfernen.

Ein gattungsgemäßes medizinisches Dilatationsinstrument ist beispielsweise aus der WO 2008/134288 A2 bekannt. Bei einer ersten Ausgestaltungsform dieses bekanten Dilatationsinstruments besteht die Werkzeugspitze aus mehreren Lamellen, die distal miteinander verbunden sind und über das Betätigungselement so gestaucht werden, dass sich die Lamellen ballonartig radial aufweiten. Diese Konstruktion ist durch aus zur Erweiterung von weichem Gewebe geeignet, jedoch knicken die Lamellen nach innen ein, wenn diese zum Entfernen knöcherner Verwachsungen eingesetzt werden.

Gemäß einer zweiten Ausführungsform dieses bekannten Dilatationsinstruments besteht die Werkzeugspitze aus mehreren Lamellen die distalseitig nicht miteinander verbunden sind und über eine Verdickung am distalen Ende des Betätigungselements radial aufbiegbar ist. Nachteilig an dieser Ausgestaltung ist, dass die Werkzeugspitze auch in der nicht aufgeweiteten Position der Lamellen der Werkzeugspitze offen ist, so dass Gewebe in die Dilatationsmechanik eindringen kann. Darüber hinaus ermöglichen die biegeelastischen Lamellen für den Benutzer nur eine bedingte taktile Kontrolle der Arbeit der Werkzeugspitze.

Weiterhin sind Instrumente zum Dilatieren von Herzgefäßen bekannt, die mittels verschwenkbarer Segmente Gefäße erweitern und Gefäßverschlüsse durchstoßen können. Diese bekannten medizinischen Instrumente zum Erweitern von Gefäßen sind jedoch nur dazu ausgelegt, geringen Kräfte zu übertragen, um eine Verletzung der zu erweiternden Gefäße zu verhindern. Zum Dilatieren knöcherner Verwachsungen, wie beispielsweise in Stirnhöhlen, sind diese bekannten Instrumente jedoch nicht geeignet.

Davon ausgehend liegt der Erfindung die **Aufgabe** zugrunde, ein Dilatations-instrument zu schaffen, das bei ausreichender Stabilität ein feinfühliges Betätigen ermöglicht.

Diese Aufgabe wird erfindungsgemäss durch das Instrument gemäss Anspruch 1 gelöst.

Durch diese Ausbildung der Werkzeugspitze mit den das distale Ende des Betätigungselements vollständig umschließenden verschwenkbaren Segmenten ist die erfindungsgemäße Werkzeugspitze in der geschlossenen Position der Segmente vollständig gegenüber der Umgebung geschlossen, wodurch auch die Dilatationsmechanik geschützt wird. Darüber hinaus lassen sich die verschwenkbaren Segmente als formstabile Schwenkelemente ausbilden, die bei großer Aufweitkraft eine feinfühlige Betätigung durch den Operateur erlauben.

Vorteilhafterweise umschließen die verschwenkbaren Segmente der Werkzeugspitze das verdickte distale Ende des Betätigungselements in der geschlossenen Position der Werkzeugspitze vollständig, jedoch können es fertigungs- und/oder toleranzbedingt auch minimale Spalten zwischen den verschwenkbaren Segmenten verbleiben, die aber für das erfindungsgemäße im Wesentlichen vollständige Umschließen des verdickten distalen Endes des Betätigungselements unschädlich sind.

Zur Ausbildung der Dilatationsmechanik wird gemäß einer bevorzugten Ausführungsform der Erfindung vorgeschlagen, dass im Inneren der von den einzelnen Segmenten gebildeten Werkzeugspitze ein Aufnahmeraum für das verdickte distale Ende des Betätigungselements ausgebildet ist, wobei die Innenkontur der einzelnen Segmente der Werkzeugspitze so ausgebildet ist, dass die Segmente bei einer Verlagerung des verdickten distalen Endes des Betätigungselements radial nach außen verschwenken. Durch die Verlagerung des vorteilhafterweise als Zug- oder Schubstange ausgebildeten Betätigungselements in Axialrichtung des Schaftes werden die verschwenkbaren Segmente der Werkzeugspitze radial nach außen gedrückt.

Der Aufnahmeraum für das verdickte distale Ende des Betätigungselements ist vorteilhafterweise sich zum proximalen Ende der Segmente hin konisch verengend ausgebildet, so dass die Segmente beim Zurückziehen des Betätigungselements in proximaler Richtung automatisch radial nach außen verschwenkt werden und das distale Ende der Werkzeugspitze in der Form einer Tulpenblüte öffnen.

Das verdickte distale Ende des Betätigungselements ist gemäß einer praktischen Ausführungsform der Erfindung kugel- oder kegelförmig ausgebildet.

Gemäß einer ersten erfindungsgemäßen Ausführungsform wird vorgeschlagen, dass die Werkzeugspitze aus vier verschwenkbaren Segmenten besteht, die jeweils um 90° versetzt zueinander am proximalen Ende der Werkzeugspitze angelenkten sind.

Gemäß einer zweiten erfindungsgemäßen Ausführungsform wird vorgeschlagen, dass die Werkzeugspitze aus drei verschwenkbaren Segmenten besteht, die jeweils um 120° versetzt zueinander am proximalen Ende der Werkzeugspitze angelenkten sind.

Um das atraumatische Einführen der Werkzeugspitze in das Operationsgebiet zu erleichtern, wird mit der Erfindung vorgeschlagen, dass die Außenkontur des distalen Endes der Segmente der Werkzeugspitze in der geschlossenen Position der Werkzeugspitze sich konisch verjüngend ausgebildet ist. Neben der konischen Ausgestaltung nur des distalen Endes der Werkzeugspitze wird mit einer alternativen Ausgestaltungsform vorgeschlagen, dass die gesamte Außenkontur der Segmente der Werkzeugspitze in der geschlossenen Position der Werkzeugspitze in mehreren Stufen zum distalen Ende hin konisch verjüngt ausgebildet ist.

Weiterhin wird mit einer praktischen Ausführungsform der Erfindung vorgeschlagen, dass die Außenseiten der Segmente der Werkzeugspitze in der geöffneten Position der Werkzeugspitze im Wesentlichen parallel zueinander verlaufend ausgebildet sind. Diese parallele Ausgestaltung der Seiten der verschwenkbaren Segmente in der geöffneten Arbeitsposition der Werkzeugspitze ist vorteilhaft, um ein Weggleiten der Werkzeugspitze in noch nicht gesichtete Bereiche des Operationsgebiets zu verhindern.

Da die Werkzeugspitze insbesondere bei der Entfernung knöchriger Verwachsungen einem stetigen Verschleiß ausgesetzt ist, wird mit der Erfindung vorgeschlagen, dass die Werkzeugspitze lösbar mit dem Schaft verbunden ist.

Die Montage und Demontage des Instruments kann erfindungsgemäß dadurch erleichtert werden, dass der in der Werkzeugspitze gelagerte Teil des Betätigungselements über einen Kopplungsmechanismus lösbar mit dem im Schaft gelagerten Teil des Betätigungselements gekoppelt ist.

Schließlich wird mit der Erfindung vorgeschlagen, dass die Werkzeugspitze mit einer elastischen Kunststoffumhüllung überziehbar ist, um das Eindringen von Gewebe in die Dilatationsmechanik zu verhindern.

Weiterhin wird mit der Erfindung vorgeschlagen, dass die verschwenkbaren Segmente der Werkzeugspitze mittels der elastischen Kunststoffumhüllung aus geöffneten Position der Werkzeugspitze zurück in die geschlossenen Position der Werkzeugspitze überführbar sind. Bei dieser Ausgestaltung wird die Rückstellkraft der elastischen Kunststoffumhüllung ausgenutzt, um nach dem Aufspreizen der verschwenkbaren Segmente und dem damit verbundenen Dehnen der Kunststoffumhüllung die Werkzeugspitze wieder in die geschlossene Position zu überführen.

Zur Ausgestaltung der elastischen Kunststoffumhüllung wird erfindungsgemäß vorgeschlagen, dass die elastische Kunststoffumhüllung als proximalseitig offener Überzug ausgebildet ist, an dessen proximalseitigem Rand ein umlaufendes Dichtelement ausgebildet ist.

Vorteilhafterweise ist das Dichtelement als umlaufende Verdickung ausgebildet ist, die in einer entsprechenden umlaufenden Nut der Werkzeugspitze festlegbar ist.

Weitere Merkmale und Vorteile der Erfindung ergeben sich anhand der zugehörigen Zeichnungen, in denen fünf Ausführungsbeispiele eines erfindungsgemäßen medizinischen Dilatationsinstruments nur beispielhaft dargestellt sind, ohne die Erfindung auf dieses Ausführungsbeispiele zu beschränken. In den Zeichnungen zeigt:
- Fig. 1: eine schematische teilweise geschnittene Seitenansicht eines erfindungsgemäßen medizinischen Dilatationsinstruments;
- Fig. 2: eine vergrößerte Darstellung des Details II, eine erste Ausführungsform einer Werkzeugspitze in der geschlossenen Position darstellend;
- Fig. 3: eine nicht geschnittene perspektivische Ansicht der Darstellung gemäß Fig. 2, jedoch die Werkzeugspitze in der geöffneten Position darstellend;
- Fig. 4: eine geschnittene Seitenansicht einer zweiten Ausführungsform einer Werkzeugspitze in der geschlossenen Position;
- Fig. 5: eine Seitenansicht der Werkzeugspitze gemäß Fig. 4, jedoch die geöffnete Position darstellend;
- Fig. 6: eine Seitenansicht einer dritten Ausführungsform einer Werkzeugspitze in der geschlossenen Position;
- Fig. 7: eine Seitenansicht einer vierten Ausführungsform einer Werkzeugspitze in der geschlossenen Position;
- Fig. 8: eine geschnittene Seitenansicht einer fünften Ausführungsform einer Werkzeugspitze in der geschlossenen Position und
- Fig. 9: eine Seitenansicht der Werkzeugspitze gemäß Fig. 8, jedoch die geöffnete Position darstellend.

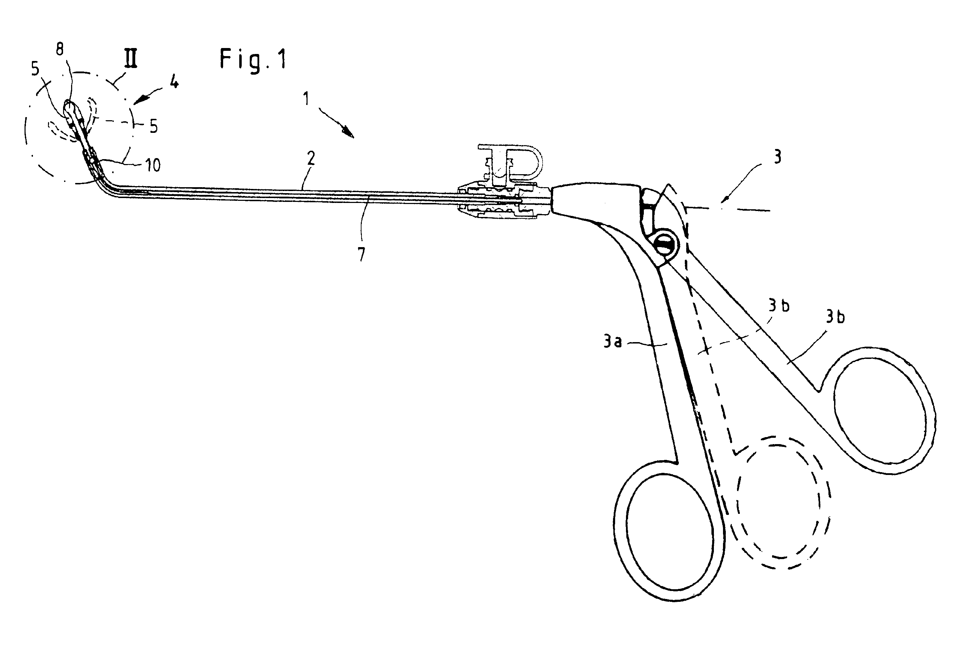

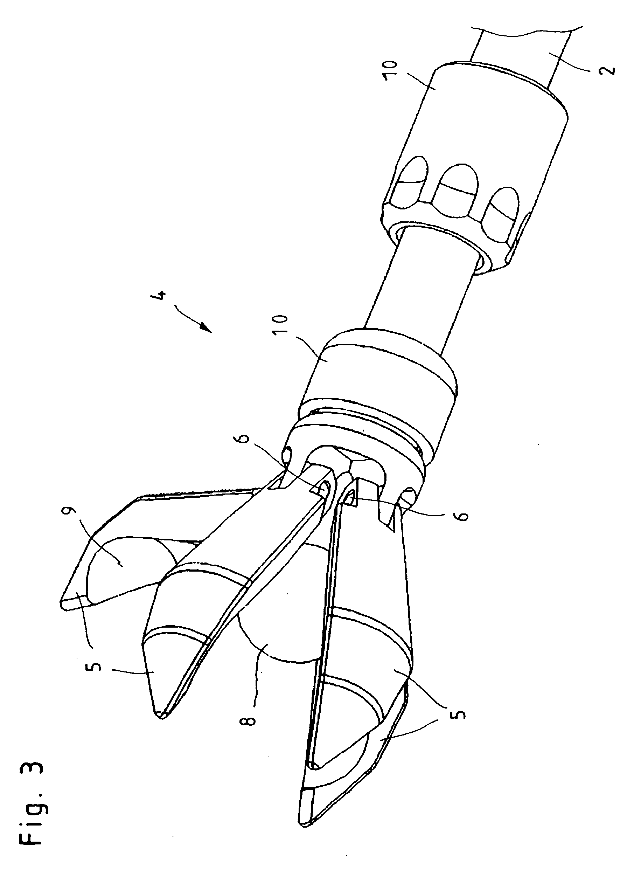

Das in der Abbildung Fig. 1 schematisch dargestellte medizinische Dilatationsinstrument 1 besteht im Wesentlichen aus einem hohlen Schaft 2, an dessen proximalem Ende eine Handhabe 3 angeordnet ist, die bei der dargestellten Ausführungsform aus einem starren Griffteil 3a und einem gegenüber dem starren Griffteil 3a verschwenkbaren Griffteil 3b besteht. Am distalen Ende des Schaftes 2 ist eine Werkzeugspitze 4 angeordnet, die wie insbesondere aus Fig. 3 ersichtlich, aus mehreren Segmenten 5 besteht, die um Schwenkachse 6 radial nach außen verschwenkbar am proximalen Ende der Werkzeugspitze 4 gelagert sind.

Wie weiterhin aus Fig. 1 ersichtlich, stehen die Werkzeugspitze 4 und der verschwenkbare Griffteil 3b der Handhabe 3 über ein in dem hohlen Schaft 2 axial verschiebbar gelagertes, als Zugstange ausgebildetes Betätigungselement 7 derart in Wirkverbindung miteinander, dass das Verstellen des Griffteils 3b der Handhabe 3 das Verschwenken der Segmente 5 der Werkzeugspitze 4 von der geschlossenen Position (durchgezogene Darstellung gemäß Fig. 1, sowie Fig. 2, 4, 6 und 7) in die geöffnete Stellung (gestrichelte Darstellung gemäß Fig. 1 sowie Fig. 3 und 5) bzw. umgekehrt bewirkt. Die jeweils zugehörige Stellung des verschwenkbaren Griffteils 3b der Handhabe 3 ist in der Abbildung Fig. 1 ebenfalls durchgezogen (für die geschlossene Position) und gestrichelt (für die geöffnete Position) dargestellt.

Alternativ zu der dargestellten Ausbildung des Betätigungselements 7 als Zugstange ist es selbstverständlich auch möglich, die Mechanik des Dilatationsinstruments 1 so auszugestalten, dass das Betätigungselement 7 als Druckstange ausbildbar ist.

Bei den dargestellten Ausführungsbeispielen besteht die Werkzeugspitze 4 jeweils aus vier Segmenten 5, die um 90° zueinander versetzt verschwenkbar am proximalen der Werkzeugspitze 4 gelagert sind. Selbstverständlich ist es auch möglich die Werkzeugspitze 4 auch aus mehr oder weniger Segmenten 5 bestehend aufzubauen, beispielsweise aus drei Segmenten 5, die um 120° zueinander versetzt verschwenkbar am proximalen der Werkzeugspitze 4 gelagert sind.

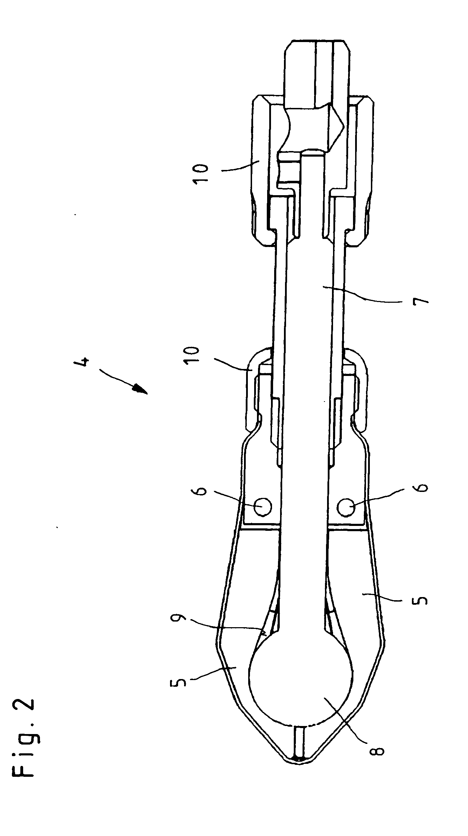

Die verschwenkbaren Segmente 5 der Werkzeugspitze 4 sind immer so angeordnet und ausgebildet, dass sie in der geschlossenen Position der Werkzeugspitze 4 eine geschlossene Außenkontur bilden und, wie aus Fig. 2 ersichtlich, das distale Ende 8 des Betätigungselements 7 im Wesentlichen vollständig umschließen.

Unter im wesentlichen vollständig umschließen ist zu verstehen, dass fertigungs- und/oder toleranzbedingt auch minimale Spalten zwischen den verschwenkbaren Segmenten verbleiben können, die aber für das geforderte Umschließen des verdickten distalen Endes des Betätigungselements unschädlich sind. Vorteilhafterweise umschließen die verschwenkbaren Segmente der Werkzeugspitze das verdickte distale Ende des Betätigungselements in der geschlossenen Position der Werkzeugspitze jedoch spaltfrei vollständig.

Zum Verschwenken der Segmente 5 in die geöffnete Position der Werkzeugspitze 4 ist einerseits das distale Ende 8 des Betätigungselements 7 verdickt kugel- oder kegelförmig ausgebildet und andererseits die Innenkontur der Segmente 5 so ausgestaltet, dass in der geschlossenen Position der Werkzeugspitze 4 im Inneren der Werkzeugspitze 4 eine Aufnahmeraum 9 für das verdickte distale Ende 8 des Betätigungselements 7 ausgebildet ist. Dieser Aufnahmeraum 9 ist bei den dargestellten Ausführungsformen sich zum proximalen Ende der Segmente 5 hin konisch verengend ausgebildet, so dass die Segmente 5 beim Zurückziehen des Betätigungselements 7 nach proximal durch das gegen die konische Verjüngung anlaufende verdickte distale Ende 8 des Betätigungselements 7 radial nach außen in die geöffnete Position der Werkzeugspitze 4 gedrückt werden.

Um das Dilatationsinstrument 1 mit der Werkzeugspitze 4 voran atraumatisch in das Operationsgebiet, beispielsweise eine Stirnhöhle, einführen zu können, ist die Außenkontur des distalen Endes der Segmente 5 der Werkzeugspitze 4 bei allen Ausführungsformen in der geschlossenen Position der Werkzeugspitze 4 sich konisch verjüngend ausgebildet.

Die in den Abbildungen Fig. 1 bis 7 dargestellten Ausführungsformen unterscheiden sich voneinander durch die Ausgestaltung der Außenkontur der Werkzeugspitze 4, die diese in der geschlossenen und/oder der geöffneten Position der Segmente 5 aufweist.

Bei der in Fig. 1 bis 3 dargestellten ersten Ausführungsform ist die Werkzeugspitze 4 doppelt konisch so ausgebildet, dass sie sich von der Schwenkachse 6 nach distal zunächst erweitert, bis sie sich zum distalen Ende der Werkzeugspitze 4 wieder konisch verjüngt.

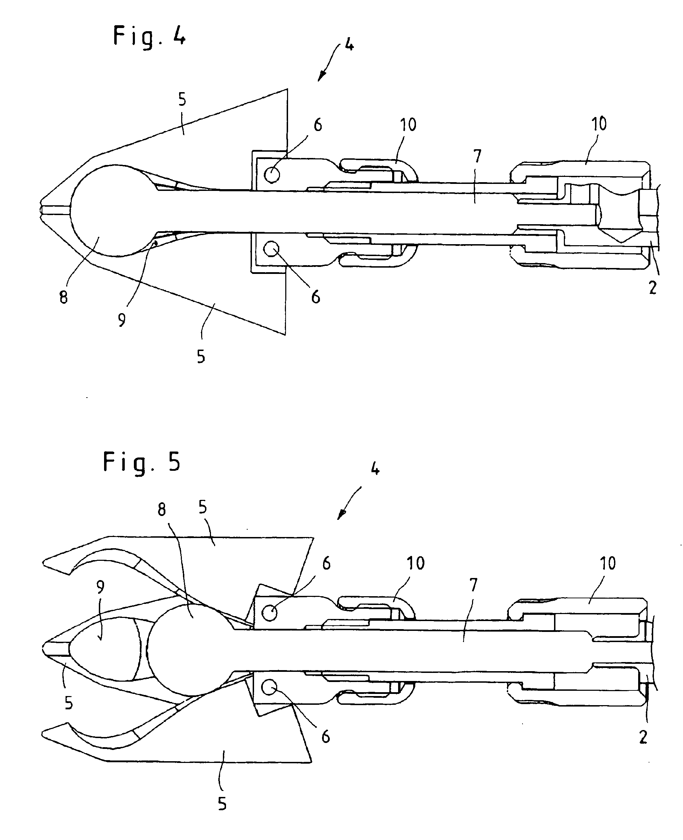

Die in den Abbildungen Fig. 4 und 5 dargestellte zweite Ausführungsform zur Ausgestaltung der Werkzeugspitze 4 unterscheidet sich von der zuvor beschriebenen Ausgestaltungsform hauptsächlich dadurch, dass die Außenseiten der Segmente 5 der Werkzeugspitze 4 in der geöffneten Arbeitsposition der Werkzeugspitze 4 im Wesentlichen parallel zueinander verlaufend ausgebildet sind.

Diese parallele Ausgestaltung der Seiten der verschwenkbaren Segmente 5 in der geöffneten Arbeitsposition ist vorteilhaft, um ein Weggleiten der Werkzeugspitze 4 in noch nicht gesichtete Bereiche des Operationsgebiets verhindern zu können, wenn die Werkzeugspitze 4 des Dilatationsinstruments 1 vom Operateur zum Weiten des Operationsgebietes und zum Brechen knöcherner Verwachsungen hin und her bewegt wird.

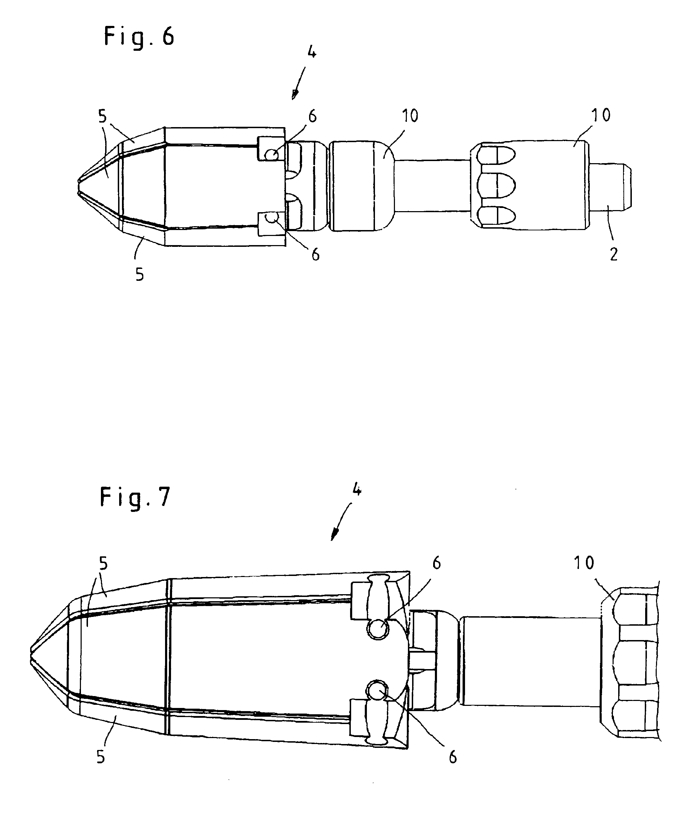

Bei der in Fig. 6 dargestellten dritten Ausführungsform sind die Außenseiten der Segmente 5 der Werkzeugspitze 4 in der geschlossenen Position der Werkzeugspitze 4 im Wesentlichen parallel zueinander verlaufend ausgebildet und verlaufen nur am distalen Ende der Werkzeugspitze sich konisch verjüngend aufeinander zu.

Die Abbildung Fig. 7 zeigt schließlich eine vierte Ausführungsform zur Ausgestaltung der Außenkontur der Werkzeugspitze 4, bei der die Außenkontur der Segmente 5 der Werkzeugspitze 4 in der geschlossenen Position der Werkzeugspitze 4 sich in mehreren Stufen zum distalen Ende hin konisch verjüngend ausgebildet ist.

Da die Werkzeugspitze 4, bzw. die verschwenkbaren Segmente 5 der Werkzeugspitze 4 im Gebrauch einem gewissen Verschleiß unterliegen, sind die dargestellten Ausführungsformen des Dilatationsinstruments 1 so ausgebildet, dass die Werkzeugspitze 4 über einen Kopplungsmechanismus 10 auswechselbar am Schaft 2 festlegbar ist.

Ebenso ist bei der in Fig. 2 dargestellten Ausführungsform der in der Werkzeugspitze 4 gelagerte Teil des Betätigungselements 7 über einen Kopplungsmechanismus 10 lösbar mit dem im Schaft 2 gelagerten Teil des Betätigungselements 7 gekoppelt. Selbstverständlich ist es auch möglich, das Betätigungselement 7 einstückig so herzustellen, dass es von distal in die geöffnete Werkzeugspitze 4 und den Schaft 2 einsetzbar ist.

Das voranstehend beschriebene Dilatationsinstrument 1 wird in der endoskopischen Chirurgie wie folgt eingesetzt:
Ausgehend von der in den Abbildungen Fig. 1 (durchgezogene Linie) sowie Fig. 2, 4, 6 und 7 dargestellten geschlossenen Position der Werkzeugspitze 4 wird das Dilatationsinstrument 1 mit der Werkzeugspitze 4 voran in das Operationsgebiet, insbesondere die Stirnhöhle eines Patienten, eingeführt, um nachfolgend verengte Bereiche aufzuweiten.

Zum Aufweiten verengter Bereiche des Operationsgebiets, wozu auch das Entfernen knöchriger Verwachsungen gehört, überführt der Operateur den verschwenkbaren Teil 3b der Handhabe in die in Fig. 1 gestrichelt dargestellte Position, wodurch das mit diesem Griffteil 3b gekoppelte Betätigungselement 7 nach proximal gezogen wird. Distalseitig bewirkt das Zurückziehen des Betätigungselements 7 nach proximal, dass die Segmente 5 der Werkzeugspitze 4 durch das verdickte distale Ende 8 des Betätigungselements 7 radial nach außen in die in den Abbildungen Fig. 1 (gestrichelte Linie) sowie Fig. 3 und 5 dargestellte geöffnete Arbeitsposition der Werkzeugspitze 4 verschwenkt werden.

Gegenüber des aus dem Stand der Technik bekannten Aufbaus der Werkzeugspitzen aus einzelnen Lamellen können bei dem zuvor beschriebenen Aufbau der Werkzeugspitze 4 die einzelnen über das verdickte distale Ende 8 des Betätigungselements 7 zu öffnenden verschwenkbaren Segmente 5 deutlich massiver und somit formstabiler ausgebildet werden, so dass auch die Spitzen der Segmente 5 zum Aufweiten und Brechen der Verwachsungen eingesetzt werden können. Durch diese Maßnahme lassen sich die Spreizoberfläche der Werkzeugspitze 4 sowie der Durchmesser der geöffneten Werkzeugspitze 4 vergrößern.

Darüber hinaus ist die mechanische Spreizkraft bei der beschriebenen Ausgestaltung der Werkzeugspitze 4 nicht von der Biegekraft einzelner Lamellen abhängig, sondern beruht rein auf der Kraftübertragung über die eingesetzten Hebelkräfte.

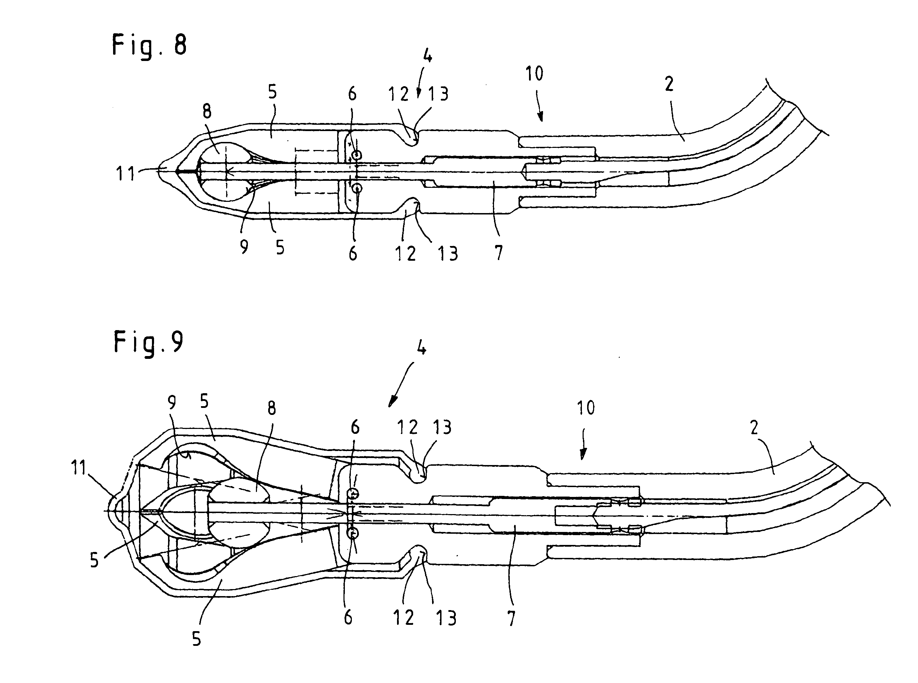

Um das Eindringen von Gewebe in die tulpenförmig geöffnete Werkzeugspitze 4 zu verhindern ist es weiterhin möglich, die Werkzeugspitze 4 mit einer elastischen Kunststoffumhüllung 11 zu überziehen, wie dies in Fig. 8 und 9 dargestellt ist. Aber auch ohne einen solchen Überzug lässt sich die beschriebene Dilatationsmechanik problemlos einsetzen, da der einfache und stabile Aufbau eine gute und schnelle Reinigung aller Bauteile ermöglicht.

Wie aus Fig. 8 und 9 ersichtlich, ist die Kunststoffumhüllung 11 als proximalseitig offener Überzug ausgebildet, der von distal über die Werkzeugspitze 4 ziehbar ist. Am proximalseitigen Ende weist die Kunststoffumhüllung 11 ein umlaufendes Dichtelement 12 auf, das abdichtend an der Werkzeugspitze 4 anliegt, um das Eindringen von Gewebe und/oder Flüssigkeit unter die Kunststoffumhüllung 11 und somit in das Innere der Werkzeugspitze 4 zu verhindern. Bei der dargestellten Ausführungsform ist das Dichtelement 12 als umlaufende wulstförmige Verdickung ausgebildet, die in einer entsprechenden umlaufenden Nut 13 der Werkzeugspitze 4 festlegbar ist.

Das Festlegen der Kunststoffumhüllung 11 in der Nut 13 hat darüber hinaus den Vorteil, dass ein Abstreifen der Kunststoffumhüllung 11 von der Werkzeugspitze 4 beim Zurückziehen des Dilatationsinstruments 1 in axialer Richtung verhindert wird.

Auch, wenn die elastische Kunststoffumhüllung 11 nur bei der in Fig. 8 und 9 dargestellten fünften Ausführungsform zeichnerisch dargestellt ist, sind derartige Kunststoffumhüllungen 11 auch bei allen anderen in den Abbildungen Fig. 2 bis Fig. 7 dargestellten und zuvor beschriebenen Ausgestaltungsformen verwendbar.

Zusätzlich zum Schutz der Werkzeugspitze 4 gegen Verunreinigungen kann die elastische Kunststoffumhüllung 11 dazu verwendet werden, die verschwenkbaren Segmente 5 der Werkzeugspitze 4 aus geöffneten Position der Werkzeugspitze 4 wieder zurück in die geschlossenen Position der Werkzeugspitze 4 zu überführen. Bei dieser Ausgestaltung wird die Rückstellkraft der elastischen Kunststoffumhüllung 11 ausgenutzt, um nach dem Aufspreizen der verschwenkbaren Segmente 5 und dem damit verbundenen Dehnen der Kunststoffumhüllung 11 die Werkzeugspitze 4 wieder in die geschlossene Position zu überführen, sobald das verdickte distale Ende 8 des Betätigungselements 7 die verschwenkbaren Segmente 5 der Werkzeugspitze 4 nicht mehr in die geöffnete Position drückt.

Ein wie zuvor beschrieben ausgestaltetes Dilatationsinstrument 1 zeichnet sich dadurch aus, dass die Dilatationsmechanik aufgrund der komplett über Hebel und Drehpunkte gesteuerten Mechanik sehr widerstandsfähig ist und darüber hinaus dem Operateur ein feinfühliges Arbeiten ermöglicht, da die über die Handhabe 3 eingeleitete Kraft im Wesentlichen spielfrei im Operationsgebiet ankommt.

### Bezugszeichenliste

- 1: Dilatationsinstrument
- 2: Schaft
- 3: Handhabe
- 3a: starrer Griffteil
- 3b: verschwenkbarer Griffteil
- 4: Werkzeugspitze
- 5: Segment
- 6: Schwenkachse
- 7: Betätigungselement
- 8: distales Ende
- 9: Aufnahmeraum
- 10: Kopplungsmechanismus
- 11: Kunststoffumhüllung
- 12: Dichtelement
- 13: Nut

## Patentansprüche

1. Medizinisches Instrument zum Dilatieren von knöchernen Strukturen, mit einem Schaft (2), an dessen distalem Ende eine Werkzeugspitze (4) angeordnet ist und an dessen proximalem Ende eine Handhabe (3) angeordnet ist, wobei die Handhabe (3) und die Werkzeugspitze (4) über ein Betätigungselement (7) derart miteinander in Wirkverbindung stehen, dass durch Betätigen der Handhabe (3) die Werkzeugspitze (4) zumindest teilweise radial erweiterbar ist und wobei das distale Ende (8) des Betätigungselements (7) verdickt ausgebildet ist, wobei die Werkzeugspitze (4) aus mehreren radial nach außen verschwenkbaren Segmenten (5) besteht, die um jeweils eine Schwenkachse (6) verschwenkbar am proximalen Ende der Werkzeugspitze (4) gelagert sind,
**dadurch gekennzeichnet,**
**dass** die radial nach außen verschwenkbaren Segmente (5) das verdickte distale Ende (8) des Betätigungselements (7) in der geschlossenen Position der Werkzeugspitze (4) vollständig umschließen, um eine geschlossene Außenkontur zu bilden.

2. Medizinisches Instrument nach Anspruch 1, **dadurch gekennzeichnet, dass** im Inneren der von den einzelnen Segmenten (5) gebildeten Werkzeugspitze (4) ein Aufnahmeraum (9) für das verdickte distale Ende (8) des Betätigungselements (7) ausgebildet ist, wobei die Innenkontur der einzelnen Segmente (5) der Werkzeugspitze (4) so ausgebildet ist, dass die Segmente (5) bei einer Verlagerung der Position des verdickten distalen Endes (8) des Betätigungselements (7) radial nach außen verschwenken.

3. Medizinisches Instrument nach Anspruch 1, **dadurch gekennzeichnet, dass** der Aufnahmeraum (9) sich zum proximalen Ende der Segmente (5) hin konisch verengend ausgebildet ist.

4. Medizinisches Instrument nach einem der Ansprüche 1 bis 3, **dadurch gekennzeichnet, dass** die Werkzeugspitze (4) aus drei oder vier verschwenkbaren Segmenten (5) besteht.

5. Medizinisches Instrument nach einem der Ansprüche 1 bis 4, **dadurch gekennzeichnet, dass** die Außenkontur des distalen Endes der Segmente (5) der Werkzeugspitze (4) in der geschlossenen Position der Werkzeugspitze (4) sich konisch verjüngend ausgebildet ist.

6. Medizinisches Instrument nach einem der Ansprüche 1 bis 5, **dadurch gekennzeichnet, dass** die Außenseiten der Segmente (5) der Werkzeugspitze (4) in der geöffneten Position der Werkzeugspitze (4) im Wesentlichen parallel zueinander verlaufend ausgebildet sind.

7. Medizinisches Instrument nach einem der Ansprüche 1 bis 5, **dadurch gekennzeichnet, dass** die Außenkontur der Segmente (5) der Werkzeugspitze (4) in der geschlossenen Position der Werkzeugspitze (4) sich in mehreren Stufen zum distalen Ende hin konisch verjüngend ausgebildet ist.

8. Medizinisches Instrument nach einem der Ansprüche 1 bis 7, **dadurch gekennzeichnet, dass** die Werkzeugspitze (4) lösbar mit dem Schaft (2) verbunden ist.

9. Medizinisches Instrument nach einem der Ansprüche 1 bis 8, **dadurch gekennzeichnet, dass** der in der Werkzeugspitze (4) gelagerte Teil des Betätigungselements (7) über einen Kopplungsmechanismus (10) lösbar mit dem im Schaft (2) gelagerten Teil des Betätigungselements (7) gekoppelt ist.

10. Medizinisches Instrument nach einem der Ansprüche 1 bis 9, **dadurch gekennzeichnet, dass** das distale Ende (8) des Betätigungselements kugel- oder kegelförmig ausgebildet ist.

11. Medizinisches Instrument nach einem der Ansprüche 1 bis 10, **dadurch gekennzeichnet, dass** die Werkzeugspitze (4) mit einer elastischen Kunststoffumhüllung (11) überziehbar ist.

12. Medizinisches Instrument nach Anspruch 11, **dadurch gekennzeichnet, dass** die verschwenkbaren Segmente (5) der Werkzeugspitze (4) mittels der elastischen Kunststoffumhüllung (11) aus geöffneten Position der Werkzeugspitze (4) zurück in die geschlossenen Position der Werkzeugspitze (4) überführbar sind.

13. Medizinisches Instrument nach Anspruch 11 oder 12, **dadurch gekennzeichnet, dass** die elastische Kunststoffumhüllung (11) als proximalseitig offener Überzug ausgebildet ist, an dessen proximalseitigem Rand ein umlaufendes Dichtelement (12) ausgebildet ist.

14. Medizinisches Instrument nach Anspruch 13, **dadurch gekennzeichnet, dass** das Dichtelement (12) als umlaufende Verdickung ausgebildet ist, die in einer entsprechenden umlaufenden Nut (13) der Werkzeugspitze (4) festlegbar ist.

## Claims

1. A medical instrument for dilating bony structures having a shaft (2) with a tool head (4) arranged at the distal end thereof and a handle (3) at the proximal end thereof, wherein the handle (3) and the tool head (4) are effectively connected to one another by means of an operating element (7) such that the tool head (4) is at least partially expandable by actuating the handle (3) and wherein the distal end (8) of the operating element (7) is thickened, wherein the tool head (4) comprises a plurality of radially outwardly pivotable segments (5), which are arranged around a pivotable axis (6) at the proximal end of the tool head (4), **characterized in that**
the radially outwardly pivotable 2 segments 2 (5) completely surround the thickened
distal end (8) of the operating element (7) in the closed position of the tool head (4) in order to form a closed outer contour.

2. The medical instrument according to claim 1, **characterized in that** a mount area (9), on the inside of the tool head (4) formed by the individual segments (5), is designed for the thickened distal end (8) of the operating element (7), wherein the inner contour of the individual segments (5) of the tool head (4) is designed such that the segments (5) pivot radially to the outside when there is a shifting of the position of the thickened distal end (8) of the operating element (7).

3. The medical instrument according to claim 1, **characterized in that** the mount area (9) is formed so as to narrow conically toward the proximal end of the segments (5).

4. The medical instrument according to any of claims 1 to 3, **characterized in that** the tool head (4) comprises three or four pivotable segments (5).

5. The medical instrument according to any of claims 1 to 4, **characterized in that** the outer contour of the distal end of the segments (5) of the tool head (4) is formed so as to taper conically, in the closed position of the tool head (4).

6. The medical instrument according to any of claims 1 to 5, **characterized in that** the outer sides of the segments (5) of the tool head (4) are formed so as to extend essentially parallel with respect to one another, in the open position of the tool head (4).

7. The medical instrument according to any of claims 1 to 5, **characterized in that** the outer contour of the segments (5) of the tool head (4) is formed so as to taper conically toward the distal end in multiple stages, in the closed position of the tool head (4).

8. The medical instrument according to any of claims 1 to 7, **characterized in that** the tool head (4) is connected to the shaft (2) in a detachable manner.

9. The medical instrument according to any of claims 1 to 8, **characterized in that** the part of the operating element (7) mounted in the tool head (4) is detachably coupled to the part of the operating element (7) arranged in the shaft (2) via a coupling mechanism (10).

10. The medical instrument according to any of claims 1 to 9, **characterized in that** the distal end (8) of the operating element is formed as a sphere or cone.

11. The medical instrument according to any of claims 1 to 10, **characterized in that** the tool head (4) can be covered with an elastic plastic sheath (11).

12. The medical instrument according to claim 11, **characterized in that** the pivotable segments (5) of the tool head (4) can be converted from the open position of the tool head (4) back to the closed position of the tool head (4) by means of the elastic plastic sheath (11).

13. The medical instrument according to either claim 11 or 12, **characterized in that** the elastic plastic sheath (11) is formed as an open cover on the proximal side, with a circumferential sealing element (12) formed on the proximal-side edge thereof.

14. The medical instrument according to claim 13, **characterized in that** the sealing element (12) is formed as a circumferential thickening, which can be affixed in a corresponding circumferential groove (13) of the tool head (4).

## Revendications

1. Instrument médical destiné à dilater des structures osseuses, comprenant un corps allongé (2) à l'extrémité distale duquel est agencée une pointe d'outil (4), et à l'extrémité proximale duquel est agencée une poignée de manoeuvre (3), instrument
dans lequel la poignée de manoeuvre (3) et la pointe d'outil (4) sont en liaison d'interaction, par l'intermédiaire d'un élément d'actionnement (7), de manière telle, que par l'actionnement de la poignée de manoeuvre (3), la pointe d'outil (4) puisse être au moins partiellement évasée ou dilatée radialement, et
dans lequel l'extrémité distale (8) de l'élément d'actionnement (7) est d'une configuration renflée, et dans lequel la pointe d'outil (4) est constituée de plusieurs segments (5) pouvant pivoter radialement vers l'extérieur, et qui sont montés à l'extrémité proximale de la pointe d'outil (4), chacun de manière à pouvoir pivoter autour d'un axe de pivotement (6),
**caractérisé**
**en ce que** les segments (5) pouvant pivoter radialement vers l'extérieur entourent totalement l'extrémité distale (8) renflée de l'élément d'actionnement (7), dans la position fermée de la pointe d'outil (4), pour former un contour extérieur fermé.

2. Instrument médical selon la revendication 1, **caractérisé en ce qu'**à l'intérieur de la pointe d'outil (4) formée par les segments (5) individuels, est formée une chambre d'accueil (9) pour l'extrémité distale .(8) renflée de l'élément d'actionnement (7), le contour intérieur des segments (5) individuels de la pointe d'outil (4) étant configuré de manière à ce que les segments (5) pivotent radialement vers l'extérieur, lors du déplacement de la position de l'extrémité distale (8) renflée de l'élément d'actionnement (7).

3. Instrument médical selon la revendication 1, **caractérisé en ce que** la chambre d'accueil (9) est d'une configuration se rétrécissant de manière conique en direction de l'extrémité proximale des segments (5).

4. Instrument médical selon l'une des revendications 1 à 3, **caractérisé en ce que** la pointe d'outil (4) est constituée de trois ou quatre segments (5) pouvant pivoter.

5. Instrument médical selon l'une des revendications 1 à 4, **caractérisé en ce que** le contour extérieur de l'extrémité distale des segments (5) de la pointe d'outil (4) est réalisé de manière à se rétrécir de façon conique, dans la position fermée de la pointe d'outil (4).

6. Instrument médical selon l'une des revendications 1 à 5, **caractérisé en ce que** les côtés extérieurs des segments (5) de la pointe d'outil (4) sont réalisés de manière à s'étendre sensiblement de façon parallèle les uns aux autres, dans la position ouverte de la pointe d'outil (4).

7. Instrument médical selon l'une des revendications 1 à 5, **caractérisé en ce que** le contour extérieur des segments (5) de la pointe d'outil (4) est réalisé de manière à se rétrécir de façon conique en plusieurs étagements vers l'extrémité distale, dans la position fermée de la pointe d'outil (4).

8. Instrument médical selon l'une des revendications 1 à 7, **caractérisé en ce que** la pointe d'outil (4) est reliée de manière amovible au corps allongé (2) de l'instrument.

9. Instrument médical selon l'une des revendications 1 à 8, **caractérisé en ce que** la partie de l'élément d'actionnement (7) montée dans la pointe d'outil (4) est couplée de manière amovible, par l'intermédiaire d'un mécanisme de couplage (10), à la partie de l'élément d'actionnement (7) montée dans le corps allongé (2) de l'instrument.

10. Instrument médical selon l'une des revendications 1 à 9, **caractérisé en ce que** l'extrémité distale (8) de l'élément d'actionnement est d'une configuration en forme de rotule sphérique ou de forme conique.

11. Instrument médical selon l'une des revendications 1 à 10, **caractérisé en ce que** la pointe d'outil (4) peut être revêtue par une housse élastique de matière plastique (11).

12. Instrument médical selon la revendication 11, **caractérisé en ce que** les segments (5) pouvant pivoter, de la pointe d'outil (4), peuvent être transférés en retour de la position ouverte de la pointe d'outil (4) à la position fermée de la pointe d'outil (4), au moyen de la housse élastique de matière plastique (11).

13. Instrument médical selon la revendication 11 ou la revendication 12, **caractérisé en ce que** la housse élastique de matière plastique (11) est réalisée sous la forme d'une housse de revêtement ouverte du côté proximal, et au niveau du bord proximal de laquelle est formé un élément d'étanchéité (12) périphérique.

14. Instrument médical selon la revendication 13, **caractérisé en ce que** l'élément d'étanchéité (12) est réalisé sous la forme d'un renflement périphérique, qui peut se fixer dans une rainure ou gorge périphérique (13) de la pointe d'outil (4).
